# EUROPEAN PATENT APPLICATION

(11) **EP 1 793 002 A1**
(43) Date of publication of application: **06.06.2007**
(21) Application number: 05768780.8
(22) Date of filing: 02.08.2005
(51) Int. Cl.: C12Q 1/66, G01N 21/78

(54) **LUCIFERASE LUMINESCENCE METHOD AND LUMINESCENT REAGENT**

(30) Priority: 05.08.2004 JP 2004229197
(71) Applicant: Toyo B-Net Co., Ltd, Tokyo 104-0031 (JP)
(72) Inventor: RYUFUKU, Masayuki, c/o TOYO B-NET CO., LTD., Chuo-ku, Tokyo 1040031 (JP); NOMURA, Ryosuke, c/o TOYO B-NET CO., LTD., Chuo-ku, Tokyo 1040031 (JP); KURITA, Akihiro, c/o TOYO B-NET CO., LTD., Chuo-ku, Tokyo 1040031 (JP)
(74) Representative: Woods, Geoffrey Corlett
(86) International application number: PCT/JP2005/014075
(87) International publication number: WO 2006/013837

(57) **Abstract**

The present invention provides a stabilized luminescence system by extending the half-life of *Coleoptera* luciferin/luciferase luminescent reactions. A luminescence method for luciferin/luciferase system, comprising reacting a luciferin with a *Coleoptera* luciferase in the presence of pyrophosphoric acid and/or a pyrophosphate to thereby generate a luminescence whose half-life is 5 minutes or more is provided. A method of using the same, luminescent reagents and luminescent reagent kits are also provided.

## Description

### TECHNICAL FIELD

The present invention relates to a method for stabilizing luminescent reactions of *Coleoptera* luciferase; a method of use of the stabilizing method; and a luminescent reagent. The luminescent reagent of the invention is applicable to any luciferase assay system, ATP assay system or luciferin assay system in which a luciferase enzyme is used as a reporter or signal.

### BACKGROUND ART

In *in vitro* luminescent reactions between *Coleoptera* luciferin/luciferase, a flash of light is observed as an emission pattern. Therefore, it has been impossible to determine such luminescent reactions precisely unless a specialized device that allows injection of a luminescent reagent is used.

To overcome this problem, a method for luciferase detection has been invented which uses a thiol reagent, such as coenzyme A (CoA) or dithiothreitol (DTT), so that the half-life of the luminescence can be extended to about five minutes with increased quantity of light emission (see Patent Document No. 1). According to this method, the quantity of light emitted from luciferase reactions can be determined precisely with a luminometer or liquid cintillation counter without an automated luminescent reagent injection device. Thus, this method has become widely utilized in reporter assay systems in which a luciferase expressed in cultured cells as a reporter is determined at high sensitivity.

However, when the reporter assay is applied to high-throughput screening (HTS) of multi-samples for drug development, the luminescence half-life of 5 minutes is not sufficient. The reagent must be added frequently and determination must be performed each time the reagent was added. Therefore, further extension of luminescence half-life has been demanded. Toward the solution of this problem, there have been invented a method in which AMP co-exists in a luciferase/luciferin reaction system (see Patent Document No. 2); and a method in which carbon dioxide is dissolved in a luciferase/luciferin reaction system (see Patent Document No. 3). Thus, a reaction system in which a luminescence half-life is extended up to 8 hours has been reported. However, in any of these methods, co-existence of expensive CoA and a high concentration (5 mM or more) of a thiol reagent such as DTT was necessary in reaction solutions. Since thiol reagents have SH-groups in their structures, they become readily degraded in aqueous solutions, in particular, when they are at high concentrations. It is known that thiol reagents oxidized or degraded by dissolved oxygen, etc. remarkably inhibit the efficiency of luciferin/luciferase reactions.

As an example of improvement of luminescent reactions without using thiol reagents such as CoA or DTT, addition of a pyrophosphate to reaction systems is known. This addition has been confirmed to enhance the intensity of luminescence (see Non-Patent Documents Nos. 4 and 5; Patent Document No. 6). However, no effect was observed on the stabilization of luminescent reactions. Further, with respect to luminescent reactions in ATP assays to determine the amount of ATP in a sample (this assay is recognized as a major use for *Coleoptera* luciferin/luciferase luminescent reactions), addition of a pyrophosphate into the reaction system did not extend the half-life of the luminescence.

There has been an invention which extends a luminescence half-life by inhibiting luminescent reactions with increased salt concentrations (see Patent Document No. 7). However, no method for extending a luminescence half-life has been found yet which is applicable to any one of luciferase assay, ATP assay and luciferin assay.
Patent Document No. 1: Japanese Patent No. 3,171,595
Patent Document No. 2: US Patent No. 5,866,348
Patent Document No. 3: US Patent No. 6,060,261
Non-Patent Document No. 4: J. Biol. Chem. 233, 1528-1537 (1958)
Non-Patent Document No. 5: Arch. Biochm. Biophys. 46, 399-416 (1953)
Patent Document No. 6: Japanese Unexamined Patent Publication No. Hei 8-47399
Patent Document No. 7: US Patent No. 6,503,723

### DISCLOSURE OF THE INVENTION

### PROBLEM FOR SOLUTION BY THE INVENTION

It is an object of the present invention to extend the half-life of luciferin/luciferase luminescent reactions and to provide a stabilized luminescent system.

### MEANS TO SOLVE THE PROBLEM

The present inventors have succeeded in extending a luminescence half-life by allowing pyrophosphoric acid or a pyrophosphate to co-exist in luciferin/luciferase luminescent reactions, and thereby succeeded in stabilizing the luminescent reactions. Thus, the present invention has been achieved.

The subject matter of the present invention is as described below.
(1) A luminescence method for luciferin/luciferase system, comprising reacting a luciferin with a *Coleoptera* luciferase in the presence of pyrophosphoric acid and/or a pyrophosphate to thereby generate a luminescence whose half-life is 5 minutes or more.
(2) The method according to (1), wherein the pyrophosphoric acid and/or the pyrophosphate is present at a concentration of 0.01 mM to 10 mM.
(3) The method according to (1), wherein the reaction system further comprises adenosine triphosphate and magnesium ions.
(4) The method according to any one of (1) to (3), wherein the reaction system further comprises an organic sulfur reagent.
(5) The method according to (4), wherein the organic sulfur reagent is present at a concentration of 10 mM or less.
(6) A method of measuring the amount of *Coleoptera* luciferase in a sample, comprising using the method according to (1).
(7) A method of measuring the amount of ATP in a sample, comprising using the method according to (1).
(8) A method of measuring the amount of luciferin in a sample, comprising using the method according to (1).
(9) A luminescent reagent to generate a luminescence by the method according to (1), comprising pyrophosphoric acid and/or a pyrophosphate.
(10) The reagent according to (9), which is used as a luciferase assay reagent for measuring the amount of *Coleoptera* luciferase in a sample.
(11) The reagent according to (9), which is used as an ATP assay reagent for measuring the amount of ATP in a sample.
(12) The reagent according to (9), which is used as a luciferin assay reagent for measuring the amount of luciferin in a sample.
(13) A luminescent reagent kit comprising the reagent according to (9).

The present inventors have elucidated the effect of pyrophosphoric acid which affects the kinetics or luminescence intensity of *in vitro* luciferin/luciferase luminescent reactions by luminous *Coleoptera,* and confirmed the possibility that pyrophosphoric acid will be a promising factor for regulating luminescent reaction systems suitable for industrial applications.

The inventors have made it clear that luminescence intensity and luminescence half-life can be regulated by using pyrophosphoric acid in luminescent reactions with firefly luciferase which is widely used in industrial applications. The inventors have also confirmed that, by using pyrophosphoric acid at an appropriate concentration, it is possible to construct a luminescent reaction system applicable to high-through put screening (HTS), which has a stable luminescence intensity and a luminescence half-life of several hours without addition of thiol reagents such as CoA or DTT used widely not only in luciferase assays but also ATP assays. It is believed that a luminescent reaction system capable of retaining a luminescent reaction for a long period of time could be constructed with the two actions of pyrophosphoric acid; these actions are to regulate the initial luminescent reaction and to promote the turnover of luciferase enzyme. However, details of this hypothesis and the action mechanism have not been elucidated yet. Further, while the presence of an organic sulfur reagent such as DTT was necessary in conventional luminescent reaction systems where CoA was present, organic sulfur reagents (sodium diethyldithiocarbamate was used in Examples) revealed no remarkable effect upon reactions in those luminescent reaction systems having a luminescence half-life of several hours enabled by pyrophosphoric acid. Further examination of the role of organic sulfur reagent in luminescent reactions systems with pyrophosphoric acid should be made in the future.

The present specification encompasses the contents of the specification and/or the drawings of Japanese Patent Application No. 2004-229197 based on which the present application claims priority.

### EFFECT OF THE INVENTION

According to the present invention, luminescent reactions excellent in duration and stability have become possible instead of conventional luciferase luminescence measuring methods characterized by speed and high sensitivity.

For example, according to the present invention, it is possible to control the half-life of the luminescence of luciferin/luciferase luminescent reactions from 5 minutes to 6 hours freely.

By adding pyrophosphoric acid and/or a pyrophosphate to a luciferin/luciferase luminescent reaction system, it is possible to extend the half-life of the luminescence more than 2-fold.

### BRIEF DESCRIPTION OF THE DRAWINGS

Fig. 1 shows the time course of luminescence intensity in luciferin/luciferase luminescent reactions using a luciferase luminescent reagent with various concentrations of potassium pyrophosphate.
Fig. 2 shows the time course of luminescence intensity in luciferin/luciferase luminescent reactions using a luciferase luminescent reagent with or without potassium pyrophosphate.
Fig. 3 shows the time course of luminescence intensity in luciferin/luciferase luminescent reactions using a luciferase luminescent reagent with or without potassium pyrophosphate and sodium diethyldithiocarbamate.
Fig. 4 shows the time course of luminescence intensity in luciferin/luciferase luminescent reactions using an ATP luminescent reagent with or without potassium pyrophosphate and sodium diethyldithiocarbamate.
Fig. 5 shows the time course of luminescence intensity in luciferin/luciferase luminescent reactions using a luciferase luminescent reagent with or without pyrophosphoric acid, potassium pyrophosphate or sodium pyrophosphate.
Fig. 6 shows the time course of luminescence intensity in luciferin/luciferase luminescent reactions using an ATP luminescent reagent with or without pyrophosphoric acid, potassium pyrophosphate or sodium pyrophosphate.

### BEST MODE FOR CARRYING OUT THE INVENTION

The luciferase used in the present invention is an enzyme that acts on a luciferin from firefly (order *Coleoptera,* family *Lampyridae),* a polyheterocyclic organic acid D-(-)-2-(6'-hydroxy-2'-benzothiazolyl)-Δ2-thiazoline-4-carboxylic acid (hereinafter, simply referred to as "luciferin" unless otherwise specified) as a luminescence substrate, and which catalyzes the oxidation of the luciferin to produce photons. The luciferase includes any enzyme that participates in luminescent reactions derived from an insect belonging to *Coleoptera,* such as those luciferases from the families *Lampyridae, Elateridae, Omethidae* and *Rhagophthalmidae.* Also included are variants of these luciferases produced by recombinant DNA techniques, mutagenesis techniques and the like so that the stability of the enzyme proteins per se or the luminescent properties thereof is artificially modified.

The concentration of luciferase in luciferin/luciferase luminescent reaction systems may be 100 femto g/ml to 100 µg/ml, preferably 1 pg/ml to 10 µg/ml, for luciferase assays. For ATP assays or luciferin assays, the concentration of luciferase may be 0.1 µg/ml to 100 µg/ml, preferably 1 µg/ml to 20 µg/ml.

The luciferin used in the present invention is a *Coleoptera* luciferin as stated above, including both of those luciferins extracted and purified directly from an insect belonging to the order *Coleoptera* and those luciferins synthesized chemically. Further, a luminescent substrate which is a *Coleoptera* luciferin derivative and has luminescence activity upon digestion of a specific enzyme is also included in the luciferin. Examples of such a *Coleoptera* luciferin derivative include, but are not limited to, 4-methyl-D-luciferin, D-luciferyl-L-methionine, 6-O-galactopyranosyl-luciferin, DEVD-luciferin, luciferin-6'methyl ester, luciferin 6'-chloroethyl ester, 6'-deoxyluciferin, and luciferin 6'-benzyl ester. Luciferin and derivatives thereof may be in a salt form. As salts, potassium salts, sodium salts and the like may be enumerated.

The concentration of luciferin in luciferin/luciferase luminescent reaction systems may be 0.001 mM to 100 mM, preferably 0.01 mM to 10 mM.

The pyrophosphoric acid and/or the pyrophosphate used in the invention is preferably water soluble. Pyrophosphoric acid, sodium pyrophosphate, potassium pyrophosphate, ammonium pyrophosphate, sodium hydrogenpyrophosphate or the like in solution may be used.

The concentration of pyrophosphoric acid and/or a pyrophosphate may be 0.0001 mM to 100 mM, preferably 0.001 mM to 10 mM.

In the luciferin/luciferase luminescent reaction of the invention, organic sulfur reagents, adenosine triphosphate or magnesium ions may be added thereto. Buffer components may also be added.

The organic sulfur reagent used in the invention is believed to have generally an effect of protecting luciferase enzyme as a reducing agent. Specific examples of organic sulfur reagents include dithiocarbamates, xanthogenates, thiophosphates, thiazoles and thiol reagents.

Dithiocarbamates include, for example, piperidine pentamethylenedithiocarbamate, pipecoline methylpentamethyldithiocarbamate, zinc dimethyldithiocarbamate, zinc diethyldithiocarbamate, zinc dibutyldithiocarbamate, zinc ethylphenyldithiocarbamate, sodium dimethyldithiocarbamate, sodium diethyldithiocarbamate, sodium dibutyldithiocarbamate, potassium dimethyldithiocarbamate, copper dimethyldithiocarbamate, iron dimethyldithiocarbamate, lead ethylphenyldithiocarbamate, selenium diethyldithiocarbamate, tellurium diethyldithiocarbamate and ammonium diethyldithiocarbamate. However, metal salts are not limited to these compounds, and salts of any metal listed in the periodic table may also be used depending on purposes.

Xanthogenates include potassium xanthogenate, zinc butylxanthogenate, sodium isopropylxanthogenate, but are not limited thereto and any xanthogenate capable of forming salts with any metal listed in the periodic table may also be used.

Thiophosphates include, for example, piperidine-bis(o,o-distearyldithiophosphate).

Thiazoles include, for example, 2-mercaptobenzothiazole, zinc 2-mercaptobenzothiazole, sodium 2-mercaptobenzothiazole, 2-mercaptobenzothiazole cyclohexylamine salt and copper 2-mercaptobenzothiazole. However, thiazoles are not limited to these compounds, and any thiazole that is capable of forming a salt or complex with any metal listed in the periodic table may also be used.

According to the present invention, it is possible to improve a luminescent reaction toward glow luminescence with suppressed flash luminescence even in a reaction system where thiol reagents such as CoA or DTT are not used, by adding pyrophosphoric acid and/or a salt thereof to the reaction system. However, thiol reagents such as DTT, dithioerythritol, β mercaptoethanol, 2-mercaptopropanol, 3-mercaptopropanol, 2,3-dithiopropanol, glutathione, CoA and the like may be used. When these thiol reagents are added to the reaction system, the concentration of CoA may be 500 µM or less, preferably 300 µM or less. The concentration of DTT may be 50 mM or less, preferably 10 mM or less.

The organic sulfur reagents described above may be used singly or in combination.

The organic sulfur reagent is used at a concentration of 0 mM to 50 mM, preferably 0.01 mM to 10 mM. It is possible to use the reagent at 2 mM or less. Alternatively, organic sulfur reagents may not be added.

Adenosine triphosphate reacts with luciferase to produce luciferyl-AMP (adenylate) from luciferin, contributing to the luminescent reaction. Adenosine triphosphate may be in a salt form. As salts thereof, disodium salt, dipotassium salt, magnesium salt and the like may be enumerated.

Adenosine triphosphate is used at a concentration of 0.01 pM to 50 mM, preferably 0.01 mM to 10 mM.

Magnesium ions are considered as a cofactor for luminescent reactions. Examples of compounds containing magnesium ions include magnesium chloride, magnesium carbonate hydroxide, magnesium sulfate, magnesium acetate, and magnesium hydrogenphosphate.

Magnesium ions are used at a concentration of 0.001 mM to 100 mM, preferably 0.1 mM to 50 mM.

Buffer components produce effects of pH stabilization and optimization of luminescent reactions. Examples of buffer components include Tris-sulfuric acid and Tricine. The kinds of buffer components may be selected depending on the type of the assay which utilizes luminescent reactions.

Buffer components are used at a concentration of 1 mM to 500 mM, preferably 5 mM to 100 mM.

The above-mentioned components make major contribution to the regulation of luminescent reactions. However, various compositions (such as surfactants or glycerol necessary for the lysis of cultured cells) may also be added to the reaction system depending on the use of the luminescent reaction.

Luciferin/luciferase luminescent reactions may be performed at 20-25°C and at pH 5.0-8.5.

In the luciferin/luciferase luminescent reaction of the invention, it is possible to regulate the luminescence half-life to be 5 minutes or more, 10 minutes or more, 30 minutes or more, 1 hour or more, or 2 hours or more (e.g., 6 hours).

In the luciferin/luciferase luminescent reaction of the invention, it is also possible to regulate and extend the half-life of the luminescence 2-fold or more, 4-fold or more, 12-fold or more, 24-fold or more, or 48-fold or more (e.g., 144-fold) as compared to luminescent reactions where pyrophosphoric acid and/or a pyrophosphate is not added.

The luciferin/luciferase luminescent reaction of the invention is applicable to determination of the amount of *Coleoptera* luciferase (luciferase assay), determination of the amount of ATP (ATP assay), determination of the amount of luciferin (luciferin assay), and so forth.

Luciferase assay is a method in which a luciferase gene is used as a reporter gene, and the luminescence activity thereof is measured to examine the transcription activity of a gene of interest. For example, a vector in which a luciferase gene is linked downstream of a promoter for a gene of interest is transfected into cultured cells, and the resultant cells are cultured further. When the transcription activity of the promoter is strong in the process of cell proliferation, a large quantity of luciferase enzyme is produced within cells. When the transcription activity is weak, the yield of luciferase enzyme decreases. With the luciferin/luciferase luminescent reaction according to the method of the invention, it is possible to determine the amount of luciferase enzyme with high sensitivity and in a simple manner. Luciferase assay is applicable to analysis of gene expression (analysis of the transcription activity of promoters and enhancers); analysis of the mode of action of mRNA in cells; elucidation of the structure and the mode of action of proteins having gene regulating function (e.g., receptors); elucidation of the organ-specific expression mode of genes in transgenic plants; and so forth.

ATP assay is a method of determining the amount of ATP. ATP is an abbreviation for adenosine triphosphate which is a high energy substance with a molecular weight of 507.2 and is involved in various metabolic activities of organisms. Organisms synthesize compounds such as ATP via metabolic passways using substances absorbed from foods. Thus, organisms store energy tentatively within their bodies in the form of ATP, which is degraded when necessary to obtain energy. It is possible to use luciferin/luciferase reactions for measuring this ATP rapidly and with high sensitivity. Luciferin/luciferase reactions are a reaction depending on the amount of ATP. Luciferin and ATP react with each other to produce luciferin adenylate. This luciferin adenylate reacts with oxygen in the presence of luciferase enzyme to undergo degradation through oxidative decarboxylation. A part of the energy generated in the process of this decarboxylation appears as a luminescent reaction. By quantitatively determining this luminescent reaction, ATP is quantitatively determined.

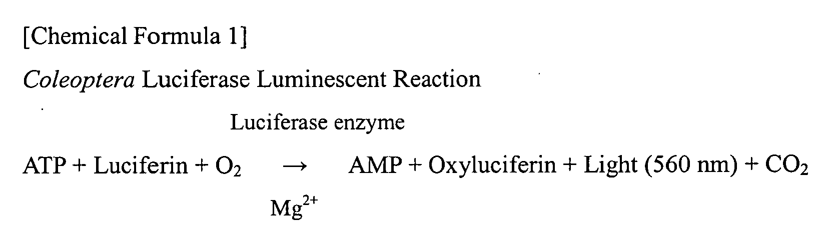

With the luciferin/luciferase luminescent reaction according to the method of the invention, it is possible to quantitatively determine ATP with high sensitivity and in a simple manner. This ATP assay is applicable to measurement of enzyme activities for such enzymes as ATPase; quantitative determination of living microorganisms; quantitative determination of living cells; and so forth.

Luciferin assay is a method of determining the amount of luminescent substrate luciferin which generates luminescence when catalyzed by luciferase. Firefly luciferin derived from *Coleoptera* is a compound with a molecular weight of 280.3 which only exists in the luminescent organ of luminous *Coleoptera.* It is widely known that this compound does not have luminescence activity when substituents located around its basic structure have been modified. Thus, 6-O-galactopyranosyl-luciferin (where galactose is linked to a substituent of luciferin) does not generate a luminescent reaction even in the presence of luciferase enzyme or a sufficient amount of ATP. However, when an enzyme reaction with β-galactosidase occurs to thereby liberate 6-O-galactopyranosyl, D-luciferin with luminescence activity is generated. By determining the yield of this D-luciferin by measuring the amount of luminescence in luciferin/luciferase luminescent reaction, it is possible to measure the enzyme activity of β-galactosidase. Since this assay uses luciferin/luciferase luminescent reaction, determination of enzyme activity with a higher sensitivity is possible, as compared to conventional methods using coloring reactions. This assay may be applicable to, for example, the measurement of kinase activity in which D-luciferin is generated from luciferin 6'-chloroethyl ester, 6'-deoxyluciferin or luciferin 6'-benzyl ester and determined; or the measurement of caspase activity with DEVD-luciferin.

As used herein, the term "luminescence half-life" means the time required for the quantity of light emitted to decrease to one half of the maximum quantity of light emitted. Luminescence half-life can be determined by measuring quantities of light emitted with the passage of time. Quantities of light emitted in luciferin/luciferase luminescent reactions may be measured by known methods. For example, luminescence intensity may be measured with a commercial luminometer.

Measurement of quantities of light emitted may be performed over a desired period at a desired time interval. For example, in luciferase assays, integrated light emission for 1 to 20 seconds may be measured from 2 seconds after the start of the luminescent reaction. In high-throughput screening (HTS) of multi-samples for drug development, the quantity of light emitted from each sample per 0.1 to 10 seconds may be measured from the start of the luminescent reaction up to 8 hours.

In ATP assays, integrated light emission for 1 to 20 seconds may be measured from 2 seconds after the start of the luminescent reaction. In high-throughput screening (HTS) of multi-samples for drug development, the quantity of light emitted from each sample per 0.1 to 10 seconds may be measured from the start of the luminescent reaction up to 5 hours.

In luciferin assays, integrated light emission for 1 to 20 seconds may be measured from 2 seconds after the start of the luminescent reaction. In high-throughput screening (HTS) of multi-samples for drug development, the quantity of light emitted from each sample per 0.1 to 10 seconds may be measured from the start of the luminescent reaction up to 5 hours.

The present invention also provides a luminescent reagent for generating luminescence in the method of the invention, comprising pyrophosphoric acid and/or a pyrophosphate. The luminescent reagent of the invention is useful in luciferase assays to determine the amount of *Coleoptera* luciferin in a sample; ATP assays to determine the amount of ATP in a sample; luciferin assays to determine the amount of luciferin in a sample; and so forth. In addition to pyrophosphoric acid and/or a pyrophosphate, the luminescent reagent of the invention may comprise luciferase, luciferin, adenosine triphosphate, organic sulfur reagents, magnesium ions, buffer components, and other components (such as surfactants or glycerol necessary for the lysis of cultured cells). These components contained in the luminescent reagent of the invention are as described above. The luminescent reagent of the invention may be in the form of an aqueous solution in which individual components are dissolved. Alternatively, the luminescent reagent of the invention may be in a freeze-dried form. The reagent in an aqueous solution form may be supplied as a frozen product, which is thawed and thoroughly brought to room temperature before use. When the luminescent reagent is freeze-dried, only those components which are relatively unstable in solution (e.g., luciferin, luciferase and adenosine triphosphate) are freeze-dried to prepare Reagent A. Each of the remaining components such as pyrophosphoric acid and/or a pyrophosphate, organic sulfur reagents, magnesium ions, buffer components, and other components (e.g., surfactants or glycerol necessary for the lysis of cultured cells) is dissolved in water to give the same concentration as that at the time of use, to thereby prepare an aqueous solution (designated Reagent B). Then, the freeze-dried product (Reagent A) may be stored frozen; and the aqueous solution (Reagent B) may be stored refrigerated or frozen. In this case, Reagent B may be thoroughly brought to room temperature and then added to Reagent A for dissolution. The reagent may be used in a state of solution.

Examples of compositions of the luminescent reagent of the invention are summarized in the following Tables.

**Table 1. Composition of Luminescent Reagent for Luciferase Assay (Luciferase Assay Reagent)**

| | Preferable Range | Suitable Range |
|---|---|---|
| Pyrophosphoric acid, pyrophosphate | 0.001 mM - 10 mM | 0.0001 mM - 100 mM |
| Luciferin | 0.01 mM - 10 mM | 0.001 mM - 100 mM |
| Adenosine triphosphate | 0.01 mM - 1 mM | 0.001 mM - 10 mM |
| Organic sulfur reagent | 0.1 mM - 10 mM | 0.01 mM - 50 mM |
| Magnesium ions | 0.1 mM - 50 mM | 0.01 mM - 100 mM |
| Buffer components | 5 mM - 100 mM | 1 mM - 500 mM |

| | | |
|---|---|---|
| Solvent: water (Milli-Q water) pH 5.0-8.5 | | |

When the luminescent reagent with the above-described composition is made into a kit, the kit may be supplied as a frozen product of one-liquid system which is prepared by freezing a solution having the above-described composition. This kit is stored at -70°C or below and, after thawing, brought to room temperature thoroughly and then used. Alternatively, a reagent kit of two-liquid system may also be supplied which comprises Reagent A (prepared by freeze-drying relatively unstable components in solution, such as luciferin, adenosine triphosphate, etc.) and Reagent B (consisting of the remaining components) to be added to Reagent A for dissolution immediately before use. In this case, the frozen product Reagent A is stored at -20°C and Reagent B is stored at 4 °C or -20°C.

**Table 2. Composition of Luminescent Reagent for ATP Assay (ATP Assay Reagent)**

| | Preferable Range | Suitable Range |
|---|---|---|
| Pyrophosphoric acid, pyrophosphate | 0.001 mM - 10 mM | 0.0001 mM - 100mM |
| Luciferin | 0.01 mM - 10 mM | 0.001 mM - 100 mM |
| Luciferase | 1 µg/mL - 20µg/mL | 0.1 µg/mL - 100 µg/mL |
| Organic sulfur reagent | 0.1 mM - 10 mM | 0.01 mM - 50 mM |
| Magnesium ions | 0.1 mM - 50 mM | 0.01 mM - 100 mM |
| Buffer components | 5 mM - 100 mM | 1 mM - 500 mM |

| | | |
|---|---|---|
| Solvent: water (Milli-Q water) pH 5.0-8.5 | | |

When the luminescent reagent with the above-described composition is made into a kit, the kit may be supplied as a frozen product of one-liquid system which is prepared by freezing a solution having the above-described composition. This kit is stored at -70°C or below and, after thawing, brought to room temperature thoroughly and then used. Alternatively, a reagent kit of two-liquid system may also be supplied which comprises Reagent A (prepared by freeze-drying relatively unstable components in solution, such as luciferin, luciferase, etc.) and Reagent B (consisting of the remaining components) to be added to Reagent A for dissolution immediately before use. In this case, the frozen product Reagent A is stored at -20°C and Reagent B is stored at 4 °C or -20°C.

**Table 3. Composition of Luminescent Reagent for Luciferin Assay (Luciferin Assay Reagent)**

| | Preferable Range | Suitable Range |
|---|---|---|
| Pyrophosphoric acid, pyrophosphate | 0.001 mM-10 mM | 0.0001 mM-100 mM |
| Luciferase | 1 µg/mL - 20 µg/mL | 0.1 µg/mL - 100 µg/mL |
| Adenosine triphosphate | 0.01 mM - 1 mM | 0.001 mM - 10 mM |
| Organic sulfur reagent | 0.1 mM - 10 mM | 0.01 mM - 50 mM |
| Magnesium ions | 0.1 mM - 50 mM | 0.01 mM - 100 mM |
| Buffer components | 5 mM - 100 mM | 1 mM - 500 mM |

| | | |
|---|---|---|
| Solvent: water (Milli-Q water) pH 5.0-8.5 | | |

When the luminescent reagent with the above-described composition is made into a kit, the kit may be supplied as a frozen product of one-liquid system which is prepared by freezing a solution having the above-described composition. This kit is stored at -70°C or below and, after thawing, brought to room temperature thoroughly and then used. Alternatively, a reagent kit of two-liquid system may also be supplied which comprises Reagent A (prepared by freeze-drying relatively unstable components in solution, such as luciferase, adenosine triphosphate, etc.) and Reagent B (consisting of the remaining components) to be added to Reagent A for dissolution immediately before use. In this case, the frozen product Reagent A is stored at -20°C and Reagent B is stored at 4 °C or -20°C.

The present invention also provides a kit comprising the luminescent reagent of the invention. The kit of the invention may contain a handling manual, a flow chart of operational procedures showing how to use the kit, and the like in addition to the luminescent reagent of the invention.

The handling manual may contain an outline of luminescent reactions and measuring principle, characteristics of the product, storage conditions, methods of preparation of reagents and operational procedures therefor, related products, trouble shooting, etc. The kit of the invention may further comprise a luciferase enzyme as an authentic sample (when the kit is used in luciferase assays); an ATP solution as an authentic sample (when the kit is used in ATP assays); a luciferin as an authentic sample (when the kit is used in luciferin assays); and the like depending on the use.

Hereinbelow, the present invention will be described more specifically with reference to the following Examples. However, these Examples are not intended to limit the scope of the present invention.

### EXAMPLE 1

A "luciferase luminescent reagent" containing 20 mM tricine, 1.07 mM (MgCO₃)₄Mg(OH)₂.5H₂O, 2.67 mM magnesium sulfate, 0.1 mM EDTA, 470 µM luciferin and 530 µM adenosine triphosphate (ATP), pH 7.8 was prepared in accordance with the formulation described in the instructions included in "PicaGene Luminescent Kit" (Toyo Ink Manufacturing Co., Ltd.; product No. PGL100), except that 270 µM CoA and 33.3 mM dithiothreitol (DTT) (thiol reagents) mentioned in the instructions were not added. To this "luciferase luminescent reagent" were added potassium pyrophosphate (Wako Purechemical Industries, Ltd.) and an organic sulfur reagent, sodium diethyldithiocarbamate (Kawaguchi Kogyo), at final concentrations of 50 µM and 2 mM, respectively, to thereby prepare a "luciferase luminescent reagent (+improvement)".

A luciferase enzyme solution (100 ng/ml) was prepared by diluting "luciferase standard enzyme (10 µg/ml)" included in "PicaGene Luminescent Kit" (Toyo Ink Manufacturing Co., Ltd.; product No. PGL100) with "PicaGene Cell Lysis Reagent LUC" (Toyo Ink Manufacturing Co., Ltd.; product No. PGC50). 10 µl of this luciferase enzyme solution was taken into a cuvette of a luminometer (Berthold, LB9506). First, 100 µl of the "luciferase luminescent reagent" prepared above was added to the enzyme solution. Immediately after the addition, the resulting mixed solution was loaded onto the luminometer, and the quantity of light emitted (RLU/sec) was measured for the initial 200 seconds at intervals of 25 seconds. Then, the quantity of light emitted was measured in the same manner using the "luciferase luminescent reagent (+improvement)". Values obtained from these measurements are shown in Table 4.

**Table 4**

| Time passed | 0 sec | 25 sec | 50 sec | 75 sec | 100 sec | 125 sec | 150 sec | 175 sec | 200 sec |
|---|---|---|---|---|---|---|---|---|---|
| Luciferase luminescent | 5,753,825 | 4,315,869 | 4,002,698 | 3,656,885 | 3,264,859 | 3,023,895 | 2,852,214 | 2,423,389 | 2,022,784 |
| reagent | 100% | 75.0% | 69.6% | 63.6% | 56.7% | 52.6% | 49.6% | 42.1% | 35.2% |
| Luciferase luminescent | 2,836,521 | 2,774,685 | 2,774,523 | 2,774,358 | 2,774,285 | 2,774,235 | 2,774,145 | 2,774,017 | 2,773,856 |
| reagent (+improvement) | 100% | 97.8% | 97.8% | 97.8% | 97.8% | 97.8% | 97.8% | 97.8% | 97.8% |

In luminescent reactions using the "luciferase luminescent reagent" without thiol reagents CoA and DTT, occurrence of flash luminescence was observed in which light emission was rapidly attenuated immediately after the addition of the reagent. The half-life of the luminescence was about 2.5 minutes. On the other hands, in luminescent reactions using the "luciferase luminescent reagent (+improvement)" containing potassium pyrophosphate and an organic sulfur reagent, occurrence of glow luminescence was observed in which nearly constant light emission was maintained for 200 seconds after a slight attenuation immediately after the addition of the reagent. The half-life of the luminescence was more than 5 minutes.

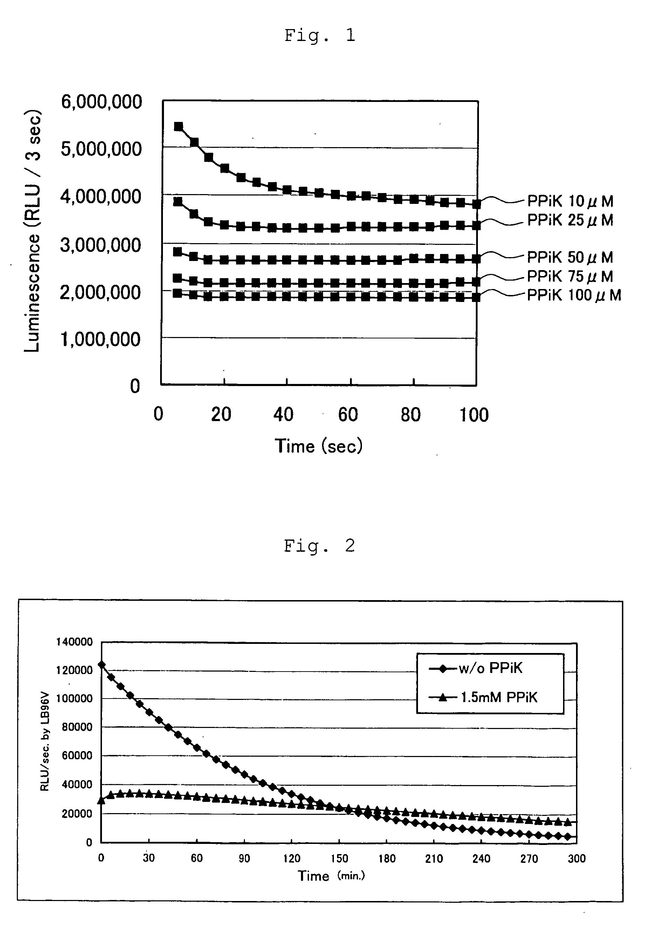

Further, to the above-described "luciferase luminescent reagent", sodium diethyldithiocarbamate was added to give a final concentration of 2 mM and potassium pyrophosphate (PPiK) was added to give a final concentration of 10 µM, 25 µM, 50 µM, 75 µM or 100 µM. Then, the above-described operations were repeated. The results are shown in Fig. 1.

When the concentration of potassium pyrophosphate was as low as 10 µM, luminescence was rapidly attenuated though the initial light emission was high. However, the luminescence half-life was extended to more than 5 minutes. On the other hand, as the concentration of potassium pyrophosphate increased, effect on stabilization of light emission tended to increase though the initial light emission decreased accordingly. When potassium pyrophosphate was added at 100 µM, the luminescence half-life was extended to more than 10 minutes. From these results, it was confirmed that the quantity of initial light emission and the stabilization of luminescence can be regulated with the concentration of potassium pyrophosphate. It is believed that these effects are attributable to the two actions of pyrophosphoric acid, i.e., the action to regulate the initial luminescent reaction in *Coleoptera* luciferin/luciferase luminescent reactions and the action to promote the turnover of luciferase enzyme.

### EXAMPLE 2

A "luciferase luminescent reagent" containing 20 mM tricine, 1.07 mM (MgCO₃)₄Mg(OH)₂.5H₂O, 2.67 mM magnesium sulfate, 0.1 mM EDTA, 33.3 mM DTT, 470 µM luciferin, 270 µM CoA and 530 µM adenosine triphosphate (ATP), pH 7.8 was prepared in accordance with the formulation described in the instructions included in "PicaGene Luminescent Kit" (Toyo Ink Manufacturing Co., Ltd.; product No. PGL100). Instead of 270 µM CoA and 33.3 mM dithiothreitol (DTT) which are the thiol reagents mentioned in the prescription, potassium pyrophosphate (Wako Purechemical Industries, Ltd.) and an organic sulfur reagent, sodium diethyldithiocarbamate, were added thereto at final concentrations of 1.5 mM and 2 mM, respectively, to thereby prepare a "luciferase luminescent reagent (+improvement)". Further, glycerol and DTT were allowed to co-exist in each of the above luminescent reagents at final concentrations of 10% and 1 mM, respectively, in order to promote the stabilization of luciferase enzyme.

A luciferase enzyme solution (100 ng/ml) was prepared by diluting "luciferase standard enzyme (10 µg/ml)" included in "PicaGene Luminescent Kit" (Toyo Ink Manufacturing Co., Ltd.; product No. PGL100) with "PicaGene Cell Lysis Reagent LUC" (Toyo Ink Manufacturing Co., Ltd.; product No. PGC50). 10 µl of this luciferase enzyme solution was dispensed into each well of a microtiter plate. Subsequently, 100 µl of the above-described luminescent reagent was added to the enzyme solution in each well. Immediately after the addition, the microtiter plate was transferred into a luminometer (Berthold; model LB96V Plus), in which the time course of the quantity of light emitted was measured for the initial 5 hours from the start of the measurement (Tables 5 and 6; Fig. 2). Since luciferin/luciferase luminescent reactions are greatly affected by temperature, all operations and measurements with a luminometer were performed in a thermostatic chamber set at 23°C.

**Table 5**

| Time passed | 0 hr | 0.5 hr | 1 hr | 2 hr | 3 hr | 4 hr | 5 hr |
|---|---|---|---|---|---|---|---|
| Luciferase | 124,053.7 | 91,562 | 62,581 | 32,564 | 12,564 | 6,754 | 432 |
| luminescent reagent | 100% | 73.8% | 50.4% | 26.2% | 10.1% | 5.4% | 0.3% |
| Luciferase | 29,876 | 33,256 | 32,487 | 27,742 | 23,568 | 20,224 | 18,544 |
| luminescent reagent (+improvement) | 100% | 111.3% | 108.7% | 92.9% | 78.9% | 67.7% | 62.1% |

**Table 6**

| | Max RLU | Max Time (min.) | Red.Ratio (RLU/min.) | Half life (min.) | Max RLU % |
|---|---|---|---|---|---|
| w/o pyrophosphoric acid | 124053.7 | 0 | 1016.7 | 61.0 | 100.0 |
| 1.5mM PPiK | 34120.3 | 18 | 51.9 | 328.7 | 27.5 |

In luminescent reactions using the "luciferase luminescent reagent", the quantity of light emitted was high from the start of the measurement. However, the luminescence was gradually attenuated, and the quantity of light emitted was decreased to 50.4% in the first 60 minutes of the measurement. Thus, the luminescence half-life was 61 minutes. On the other hands, in luminescent reactions using the "luciferase luminescent reagent (+improvement)", the quantity of light emitted (RLU/sec) was stabilized at a constant level 10 minutes after the start of the measurement and eventually attenuated gradually. Even at 5 hours from the start of the measurement, the quantity of light emitted remained at a high level, i.e., 62.1% of the maximum quantity of light emitted. The luminescence half-life was 5.5 hours.

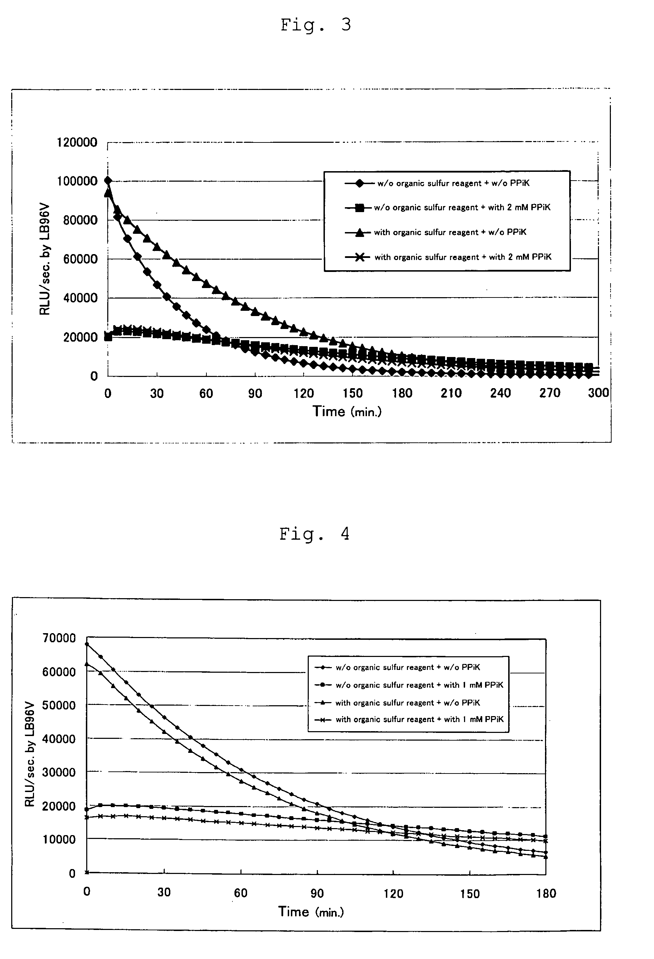

Further, four types of the above-described "luciferase luminescent reagent" were prepared: (1) w/o sodium diethyldithiocarbamate and w/o potassium pyrophosphate; (2) with potassium pyrophosphate (final concentration: 2 mM); (3) with sodium diethyldithiocarbamate (final concentration: 2 mM); and (4) with sodium diethyldithiocarbamate (final concentration: 2 mM) and with potassium pyrophosphate (final concentration: 2 mM). Then, the above-described operations were repeated. The results are shown in Table 7 and Fig. 3. It should be noted that 10% glycerol was allowed to co-exist in these experimental systems in order to stabilize luciferase enzyme, but no DTT was added for strict comparison of the effects of organic sulfur reagents.

**Table 7**

| | Max RLU | Max Time (min.) | Half life (min.) | Max RLU% |
|---|---|---|---|---|
| w/o organic sulfur reagent + w/o PPiK | 100436.8 | 0 | 28.1 | 100.0 |
| w/o organic sulfur reagent + with 2 mM PPiK | 23210.5 | 12 | 187.1 | 23.1 |
| with organic sulfur reagent + w/o PPiK | 94160.3 | 0 | 50.9 | 93.8 |
| with organic sulfur reagent + with 2 mM PPiK | 24373.3 | 12 | 169.9 | 24.3 |

It was recognized that addition of 2 mM sodium pyrophosphate suppressed the increase of initial luminescence and rapid attenuation thereof to thereby extend the luminescence half-life remarkably. When sodium pyrophosphate was not added, the luminescence half-life in systems without organic sulfur reagent was 28 minutes; and even in systems with organic sulfur reagent, the luminescence half-life was around 50 minutes. On the other hand, when sodium pyrophosphate was added, the luminescence half-life was greatly extended. It was 187 minutes in the absence of organic sulfur reagent and 169 minutes in the presence of organic sulfur reagent. In addition, stabilization of luminescent reactions was confirmed. It was confirmed that the effect on this stabilization became remarkable by addition of potassium pyrophosphate.

When light emission in luciferase assay in the presence of a thiol reagent such as CoA is measured per several ten seconds, organic sulfur reagents such as sodium diethyldithiocarbamate showed a remarkable effect in enhancing luminescence intensity and stabilizing luminescence kinetics. However, in reaction systems which do not contain thiol reagents such as CoA and in which luminescence duration time is extended to several hours, organic sulfur reagents only showed some effect in suppressing attenuation of luminescence and maintaining luminescence intensity. It was confirmed that pyrophosphoric acid has a far greater effect of contributing to the stabilization of luminescence. It is presumed that while pyrophosphoric acid *per se* acts on the regulation of *Coleoptera* luciferin/luciferase luminescent reactions directly, organic sulfur reagents play an auxiliary role therein.

### EXAMPLE 3

ATP luminescent reagent (10 ml; containing 1 mM DTT) included in "ATP Measuring Reagent Kit" (Toyo Ink Manufacturing Co., Ltd.; product No. LL100-1) was prepared. To a 5 ml aliquot of this reagent, potassium pyrophosphate (PPiK) (Wako Purechemical Industries, Ltd.) was added to give a final concentration of 1 mM. Subsequently, ATP standard reagent (ATP concentration = 1 x 10⁻¹² M) included in the kit was dispensed into a microtiter plate at 100 µl/well. The microtiter plate was immediately transferred into a luminometer (Berthold; model LB96V Plus), in which the time course of the quantity of light emitted was measured for the initial 5 hours from the start of the measurement (Table 8). Since luciferin/luciferase luminescent reactions are greatly affected by temperature, all operations and measurements with a luminometer were performed in a thermostatic chamber set at 23°C. The same operations were performed on ATP luminescent reagent without potassium pyrophosphate.

**Table 8**

| Time passed | 0 hr | 0.5 hr | 1 hr | 2 hr | 3 hr | 4 hr | 5 hr | 6 hr |
|---|---|---|---|---|---|---|---|---|
| ATP luminescent | 887,689 | 226,334 | 113,876 | 52,146 | 11,235 | 5,223 | 1,125 | 201 |
| reagent | 100% | 25.5% | 12.8% | 5.9% | 1.3% | 0.6% | 0.1% | 0.0% |
| ATP luminescent | 189,865 | 176,532 | 165,325 | 152,365 | 135,886 | 123,542 | 95,521 | 83,625 |
| reagent (1 mM PPiK) | 100% | 93.0% | 87.1% | 80.2% | 71.6% | 65.1% | 50.3% | 44.0% |

In luminescent reactions using ATP luminescent reagent without potassium pyrophosphate, the quantity of light emitted was high from the start of the measurement but rapidly attenuated. The quantity of light emitted was decreased to 45% in the first 15 minutes of the measurement. Thus, the luminescence half-life was 11 minutes. On the other hands, in luminescent reactions using ATP luminescent reagent with potassium pyrophosphate at a final concentration of 1 mM, the quantity of light emitted (RLU/sec) was stabilized at a constant level 5 minutes after the start of the measurement and eventually attenuated gradually. Even at 5 hours from the start of the measurement, the quantity of light emitted remained at a high level, i.e., 50.3% of the maximum quantity of light emitted. The luminescence half-life was about 5 hours.

Further, four types of the above-described ATP luminescent reagent were prepared: (1) w/o sodium diethyldithiocarbamate and w/o potassium pyrophosphate; (2) with potassium pyrophosphate (final concentration: 1 mM); (3) with sodium diethyldithiocarbamate (final concentration: 2 mM); and (4) with sodium diethyldithiocarbamate (final concentration: 2 mM) and with potassium pyrophosphate (final concentration: 1 mM). Then, the above-described operations were repeated. The results are shown in Table 9 and Fig. 4.

**Table 9**

| | Max RLU | Max Time(min.) | Red.Ratio (RLU/min.) | Half life (min.) | Max RLU % |
|---|---|---|---|---|---|
| w/o organic sulfur reagent + w/o PPiK | 67976.75 | 0 | 632.9 | 53.7 | 100.0 |
| w/o organic sulfur reagent + with 1 mM PPiK | 20005.5 | 10 | 50.6 | 207.8 | 29.4 |
| with organic sulfur reagent + w/o PPiK | 62036.75 | 0 | 616.1 | 50.3 | 91.3 |
| with organic sulfur reagent + with 1 mM PPiK | 16828.5 | 15 | 40.7 | 221.5 | 24.8 |

It was recognized that that addition of 1 mM sodium pyrophosphate suppressed the increase of initial luminescence and rapid attenuation thereof to thereby extend the luminescence half-life remarkably. Sodium diethyldithiocarbamate was also added to the reaction system as an organic sulfur reagent. However, sodium diethyldithiocarbamate showed no effect on the stabilization of luminescent reactions, though some decrease in light emission was observed.

To date, no regulatory factors such as thiol reagents, carbon dioxide, AMP that demonstrated effect in luminescent reaction systems in luciferase assay have produced any effect on extension of luminescence half-lives or stabilization of luminescence kinetics in luminescent reaction systems in ATP assay. On the other hand, it was confirmed that addition of pyrophosphoric acid to reaction systems produces effect of suppressing the initial luminescent reaction and extending the luminescence half-life remarkably. It is believed that stabilization of luminescent reactions was achieved also in luminescent reaction systems in ATP assay as a result of the effect of pyrophosphoric acid upon suppression of the initial luminescence and upon promotion of duration of the luminescent reaction by improvement of the turnover of luciferase enzyme.

### EXAMPLE 4

A "luciferase luminescent reagent" containing 20 mM tricine, 1.07 mM (MgCO₃)₄Mg(OH)₂.5H₂O, 2.67 mM magnesium sulfate, 0.1 mM EDTA, 470 µM luciferin and 530 µM adenosine triphosphate (ATP), pH 7.8 was prepared in accordance with the formulation described in the instructions included in "PicaGene Luminescent Kit" (Toyo Ink Manufacturing Co., Ltd.; product No. PGL100), except that 270 µM CoA and 33.3 mM DTT (thiol reagents) mentioned in the instructions were not added. To this "luciferase luminescent reagent" were added sodium pyrophosphate (Wako Purechemical Industries, Ltd.) and an organic sulfur reagent, sodium diethyldithiocarbamate, both at a final concentration of 2 mM, to thereby prepare a "luciferase luminescent reagent (+improvement)". In both reagents thus prepared, glycerol (final concentration: 10%) was allowed to co-exist in order to promote stabilization of luciferase enzyme.

A luciferase enzyme solution (100 ng/ml) was prepared by diluting "luciferase standard enzyme (10 µg/ml)" included in "PicaGene Luminescent Kit" (Toyo Ink Manufacturing Co., Ltd.; product No. PGL100) with "PicaGene Cell Lysis Reagent LUC" (Toyo Ink Manufacturing Co., Ltd.; product No. PGC50). 10 µl of this luciferase enzyme solution was taken into a cuvette of a luminometer (Berthold, LB9506). First, 100 µl of the "luciferase luminescent reagent" prepared above was added to the enzyme solution. Immediately after the addition, the resulting mixed solution was loaded onto the luminometer, and the quantity of light emitted (RLU/sec) was measured for the initial 200 seconds at intervals of 25 seconds. Then, the quantity of light emitted was measured in the same manner using the "luciferase luminescent reagent (+improvement)".

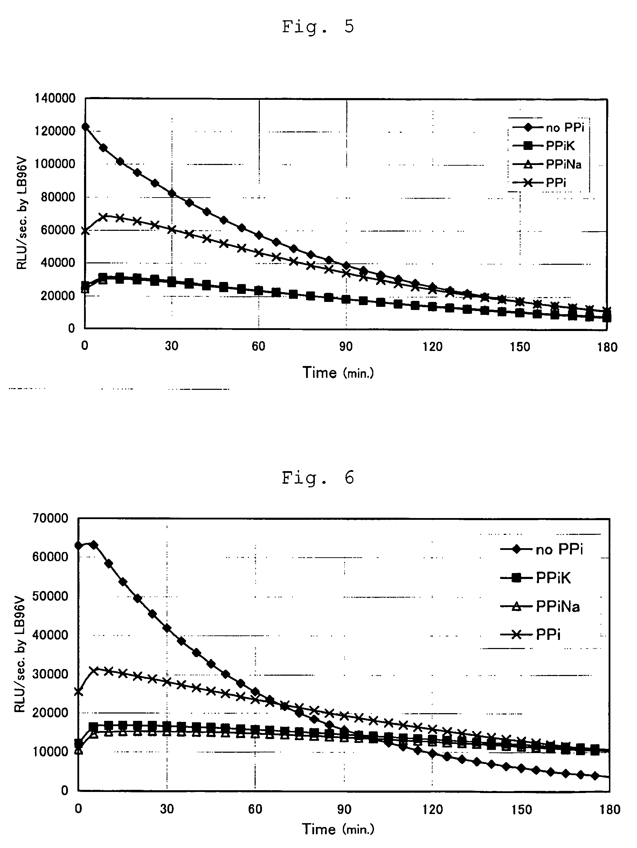

Further, another "luciferase luminescent reagent (+improvement)" was prepared by adding potassium pyrophosphate (Wako Purechemical Industries, Ltd.) or pyrophosphoric acid (Wako Purechemical Industries, Ltd.) at a final concentration of 2 mM instead of sodium pyrophosphate used in the above "luciferase luminescent reagent (+improvement)". Then, the above-described operations were repeated. The results are shown in Table 10 and Fig. 5.

**Table 10**

| | Max RLU | Max Time(min.) | Half life(min.) | Max RLU% |
|---|---|---|---|---|
| no PPi | 122651.0 | 0 | 45.8 | 100.0 |
| PPiK | 31448.0 | 12 | 122.4 | 25.6 |
| PPiNa | 30290.8 | 12 | 126.0 | 24.7 |
| PPi | 67904.5 | 6 | 111.1 | 55.4 |

In luminescent reactions using the "luciferase luminescent reagent", the quantity of light emitted was high from the start of the measurement. However, the luminescence was rapidly attenuated, and the quantity of light emitted was decreased to 47% in the first 60 minutes of the measurement. Thus, the luminescence half-life was about 46 minutes. On the other hands, in luminescent reactions using the "luciferase luminescent reagent (+improvement)" with pyrophosphoric acid or a pyrophosphate, the quantity of light emitted (RLU/sec) reached the maximum value 6 to 12 minutes after the start of the measurement, and was stabilized and gradually attenuated. In the system with pyrophosphoric acid, the maximum quantity of light emitted was 55.4% relative to the maximum quantity in the system without pyrophosphoric acid, but the luminescence half-life was extended to 111 minutes. In the systems with a pyrophosphate, the maximum quantity of light emitted was reduced to 24 to 26% relative to the maximum quantity in the system without pyrophosphoric acid, but luminescence half-lives of more than 2 hours and stable luminescent reactions were achieved.

Compared to pyrophosphoric acid (PPi), pyrophosphates PPiK and PPiNa used at the same concentration (2 mM) suppressed luminescence more strongly. It seems that this is attributable to difference in efficiency of dissociation of pyrophosphoric acid ions in aqueous solutions. It is believed that any compound which liberates pyrophosphoric acid ions into reaction solutions has effect of regulating luminescent reactions in luciferase assay, though the intensity of the effect may vary depending on the dissociation constant.

### EXAMPLE 5

ATP luminescent reagent (10 ml; containing 1 mM DTT) included in "ATP Measuring Reagent Kit" (Toyo Ink Manufacturing Co., Ltd.; product No. LL100-1) was prepared. To a 5 ml aliquot of this reagent, pyrophosphoric acid (Wako Purechemical Industries, Ltd.) was added to give a final concentration of 1 mM. Subsequently, ATP standard reagent (ATP concentration = 1 x 10⁻¹² M) included in the kit was dispensed into a microtiter plate at 100 µl/well. The microtiter plate was immediately transferred into a luminometer (Berthold; model LB96V Plus), in which the time course of the quantity of light emitted was measured for the initial 5 hours from the start of the measurement. Since luciferin/luciferase luminescent reactions are greatly affected by temperature, all operations and measurements with a luminometer were performed in a thermostatic chamber set at 23°C. The same operations were performed on ATP luminescent reagent without pyrophosphoric acid. Further, ATP luminescent reagent to which potassium pyrophosphate or sodium pyrophosphate was added at a final concentration of 1 mM instead of pyrophosphoric acid was also prepared, and the above-described operations were performed. The results are shown in Table 11 and Fig. 6.

**Table 11**

| | Max RLU | Max Time(min.) | Half life(min.) | Max RLU% |
|---|---|---|---|---|
| no PPi | 63116.8 | 5 | 42.3 | 100.0 |
| PPiK | 16800.3 | 15 | 243.5 | 26.6 |
| PPiNa | 15275.5 | 25 | 262.7 | 24.2 |
| PPi | 30874.3 | 5 | 136.5 | 48.9 |

In luminescent reactions using ATP luminescent reagent without pyrophosphoric acid, the quantity of light emitted reached the maximum value 5 minutes after the start of the measurement but was rapidly attenuated. The quantity of light emitted decreased to 66.6% in the first 30 minutes of the measurement. Thus, the luminescent half-life was about 42 minutes. On the other hands, in luminescent reactions using ATP luminescent reagent with pyrophosphoric acid at a final concentration of 1 mM, though the initial luminescence is suppressed to 50% or less relative to the initial luminescence when the "ATP luminescent reagent" was used, the quantity of light emitted (RLU/sec) reached the maximum value 5 minutes after the start of the measurement and was eventually attenuated gradually. The luminescence half-life was extended to 136.5 minutes. In the reaction systems to which a pyrophosphate was added, the maximum quantity of light emitted was suppressed to 24 to 26% relative to the maxim quantity in the reaction system without pyrophosphoric acid, but the luminescence half-lives were extended remarkably. The luminescence half-life was 243.5 minutes when the potassium salt was used, and 262.7 minutes when the sodium salt was used.

Compared to pyrophosphoric acid (PPi), pyrophosphates PPiK and PPiNa used at the same concentration (1 mM) suppressed luminescence more strongly. It seems that this is attributable to difference in efficiency of dissociation of pyrophosphoric acid ions in aqueous solutions. It is believed that any compound which liberates pyrophosphoric acid ions into reaction solutions has effect of regulating luminescent reactions in ATP assay, though the intensity of the effect may vary depending on the dissociation constant.

All publications, patents and patent applications cited herein are incorporated herein by reference in their entirety.

### INDUSTRIAL APPLICABILITY

The luciferin/luciferase luminescence method of the invention is applicable to luciferase assays, ATP assays, luciferin assays, and so forth. Therefore, by using the luciferin/luciferase luminescence method of the invention, it is possible to perform measurement of the transcription activities of genes; measurement of enzyme activities for such enzymes as ATPase; quantitative determination of living microorganisms; quantitative determination of living cells; measurement of enzyme activities for such enzymes as caspase and β galactosidase; toxicity test of drugs against cells; and so forth correctly and in HTS which handles a large quantity of samples, in a simple manner.

## Claims

1. A luminescence method for luciferin/luciferase system, comprising reacting a luciferin with a *Coleoptera* luciferase in the presence of pyrophosphoric acid and/or a pyrophosphate to thereby generate a luminescence whose half-life is 5 minutes or more.

2. The method according to claim 1, wherein the pyrophosphoric acid and/or the pyrophosphate is present at a concentration of 0.01 mM to 10 mM.

3. The method according to claim 1, wherein the reaction system further comprises adenosine triphosphate and magnesium ions.

4. The method according to any one of claims 1 to 3, wherein the reaction system further comprises an organic sulfur reagent.

5. The method according to claim 4, wherein the organic sulfur reagent is present at a concentration of 10 mM or less.

6. A method of measuring the amount of *Coleoptera* luciferase in a sample, comprising using the method according to claim 1.

7. A method of measuring the amount of ATP in a sample, comprising using the method according to claim 1.

8. A method of measuring the amount of luciferin in a sample, comprising using the method according to claim 1.

9. A luminescent reagent to generate a luminescence by the method according to claim 1, comprising pyrophosphoric acid and/or a pyrophosphate.

10. The reagent according to claim 9, which is used as a luciferase assay reagent for measuring the amount of *Coleoptera* luciferase in a sample.

11. The reagent according to claim 9, which is used as an ATP assay reagent for measuring the amount of ATP in a sample.

12. The reagent according to claim 9, which is used as a luciferin assay reagent for measuring the amount of luciferin in a sample.

13. A luminescent reagent kit comprising the reagent according to claim 9.
